(19) Europäisches Patentamt
European Patent Office
Office européen des brevets

(11) EP 2 882 730 B1

# (12) EUROPEAN PATENT SPECIFICATION

(45) Date of publication and mention of the grant of the patent:
**21.09.2016 Bulletin 2016/38**

(21) Application number: **13748031.5**

(22) Date of filing: **12.08.2013**

(51) Int Cl.:
***C07D 311/72*** *(2006.01)*

(86) International application number:
**PCT/EP2013/066820**

(87) International publication number:
**WO 2014/023849 (13.02.2014 Gazette 2014/07)**

## (54) TOCOPHEROLS WITH INCREASED COLOR STABILITY

TOCOPHEROLE MIT ERHÖHTER FARBSTABILITÄT

TOCOPHÉROLS AYANT UNE STABILITÉ DE COULEUR AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.08.2012 EP 12180088**

(43) Date of publication of application:
**17.06.2015 Bulletin 2015/25**

(73) Proprietor: **DSM IP Assets B.V.**
**6411 TE Heerlen (NL)**

(72) Inventors:
- **WILDERMANN, Angela**
  **CH-4002 Basel (CH)**
- **EBNER, Niklas**
  **CH-4002 Basel (CH)**
- **STAEUBLE, Viktor**
  **CH-4002 Basel (CH)**
- **WEYLAND, Andreas**
  **CH-4002 Basel (CH)**
- **DOORNENBAL, Otto**
  **CH-4002 Basel (CH)**

(74) Representative: **Dux, Roland**
**DSM Nutritional Products Ltd**
**Patent Department**
**Wurmisweg 576**
**4303 Kaiseraugst (CH)**

(56) References cited:
**WO-A1-91/17985** **FR-A- 962 797**
**US-A- 4 101 673**

- **ROSSI M ET AL: "The effect of bleaching and physical refining on color and minor components of palm oil", JOURNAL OF THE AMERICAN OIL CHEMISTS' SOCIETY, vol. 78, no. 10, October 2001 (2001-10), pages 1051-1055, XP002689679, ISSN: 0003-021X**
- **KEITO BOKI: "BLEACHING OF ALKALI-REFINED VEGETABLE OILS WITH CLAY MINERALS", JOURNAL OF THE AMERICAN OIL CHEMISTS' SOCIETY, SPRINGER, DE, vol. 69, no. 3, 1 March 1992 (1992-03-01), pages 232-236, XP000245388, ISSN: 0003-021X, DOI: 10.1007/BF02635892**
- **SABA NAZ ET AL: "Changes of Total Tocopherol and Tocopherol Species During Sunflower Oil Processing", JOURNAL OF THE AMERICAN OIL CHEMISTS' SOCIETY, vol. 88, no. 1, 1 January 2011 (2011-01-01), pages 127-132, XP055048035, ISSN: 0003-021X, DOI: 10.1007/s11746-010-1652-4 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 882 730 B1

## Description

### Technical Field

The invention relates to the field of preparation and use of tocopherols.

### Background of the invention

Tocopherols are important substances in the field of food and feed ingredients. It is already known for a long time that tocopherols are part of vitamin E and show an important bioactivity.

Tocopherols are known to be sensitive to air and light. It has been observed that tocopherols gradually change their color and get darker and get more yellowish / brownish upon storage at air. In certain applications this color change is disturbing the products aspect and, hence, it is desired to increase the color stability. In order to reduce this effect tocopherols are recommended to be stored in a closed vessel, particularly under an inert gas and at low temperature.

Vegetable oils and fats often have undesired strong odors and colors and, hence, refinement is necessary to have an acceptable quality for the consumption by animals or humans. This refinement involves different purification steps of which bleaching is the most prominent step. It is known since a long time that bleaching earths are efficient in the refinement of edible oils and fats. Bleaching earths are also known as bleaching clays. It was found that for certain purposes, bleaching earths may be modified by the treatment with strong acids and that those acid-treated bleaching earths, which are also known as acid activated bleaching earths, exhibit a better performance in the bleaching. However, it is known, for example from J. Am. Oil Chem. Soc (2011) 88: 127-132, that the level of tocopherols being present in the oil to be refined is remarkably reduced by the bleaching step and that the adsorption of the tocopherols on the bleaching clays is regarded as reason for this. Therefore, tocopherols are often added to edible oils and fats to increase the level of tocopherols again in order to stabilize those oils against further oxidative deterioration such as disclosed in US 4,101,673.

FR 962 797 discloses that tocopherols can be obtained from different vegetable oils. It further discloses a method of purifying and/or separation of tocopherols from such sources by means of an adsorbent being clay, kaolin or silica gel. In this method first the tocopherol is adsorbed from a highly diluted solution in a non-polar solvent onto the adsorbent, preferably in a column, and, hence, is separated from the rest of the sourcing composition. In a second step the adsorbed tocopherol is then extracted from the adsorbent by percolating by means of another solvent being more polar. By using different solvents alpha and gamma tocopherols can be separated from each other and isolated from a mixture or concentrate as they result from natural vegetable oil sources. However, this purification is very expensive as it requires a large amount of adsorbent as well as of different solvents and is not suitable for being used for large quantities and highly concentrated forms of tocopherols as they result from chemical synthesis.

The need for tocopherol on the world's market is extremely large, and, therefore, the major part of commercially available tocopherols is synthesized from petrochemicals and is not originating from bio-sources. These syntheses yield tocopherol in neat form or in very highly concentrated solutions having a limited color stability.

### Summary of the invention

The problem to be solved by the present invention is to increase the color stability of tocopherols.

Surprisingly, it has been found that the method according to claim 1 is able to solve this problem. Particularly contacting the tocopherol with acid activated bleaching earth leads to a remarkably enhancement of the color stability.

It is particularly surprising that exactly acid activated bleaching earths are suitable for this purpose in consideration of the fact that it is known that such adsorbing material exactly adsorbs in preference the product to be treated, i.e. the tocopherols.

The method is very efficient and easy. It allows producing tocopherols in high quality, thus, this method is very attractive to producers of vitamin E and to the market. It was particularly found that the color stability can be increased without the need of an additive being incorporated continuously into the tocopherol. After the contacting of the tocopherol the acid activated bleaching earth can be removed completely and, nevertheless, the tocopherol is afterwards much more color stable than the untreated tocopherol. It has been observed that particularly the increase of the red color component (increase of a* value (CIE-$L^*a^*b^*$) known in tocopherols is almost inexistent when the present method is used. Particularly the method does not involve any toxic compounds for stabilizing which would question the use of such a treated tocopherol in the field of food or feed application. Particular surprising is that the contacting of tocopherol with acid activated bleaching earth has almost no effect on the initial color, but has a remarkable beneficial effect on the color stability on the long term storage of tocopherol, i.e. in the time <u>after</u> the contact with the acid activated bleaching earth.

Further aspects of the invention are subject of further independent claims. Particularly preferred embodiments are subject of dependent claims.

## Detailed description of the invention

The present invention relates in a first aspect to a method of preparing tocopherol of formula (I) with an increased color stability comprising the step of contacting tocopherol of formula (I), in the form of neat tocopherol of formula (I) or in the form of a solution comprising more than 60 % by weight of tocopherol of formula (I), with an acid activated bleaching earth.

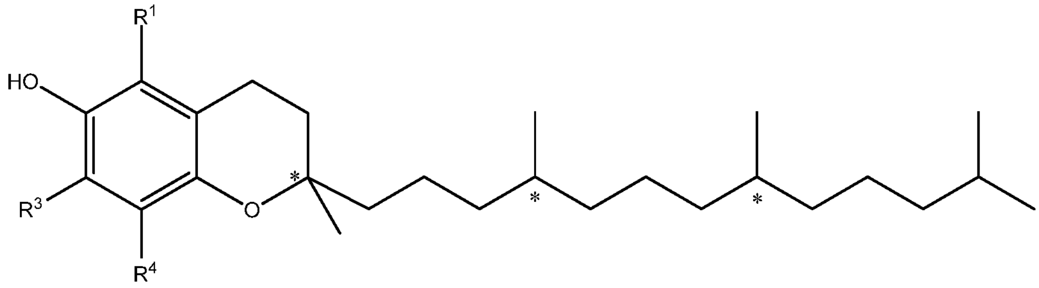

(I)

wherein $R^1$, $R^3$ and $R^4$ are independently from each other hydrogen or methyl groups;
and wherein each * represents an individual chiral centre.

The term "independently from each other" in this document means, in the context of substituents, moieties, or groups, that identically designated substituents, moieties, or groups can occur simultaneously with a different meaning in the same molecule.

A "$C_{x-y}$-alkyl" or "$C_{x-y}$-acyl" group is an alkyl or an acyl group, respectively, comprising x to y carbon atoms, i.e., for example, a $C_{1-3}$-alkyl group is an alkyl group comprising 1 to 3 carbon atoms. The alkyl group or the acyl group can be linear or branched.

The term "hydrogen" means in the present document H and not $H_2$.

The tocopherol of formula (I) is contacted with an acid activated bleaching earth.

<u>Acid activated bleaching earth</u>

The term "bleaching earth" (German: "Bleicherde") is known to the person skilled in the art to be a collective name for a group of aluminium and/or magnesium silicates comprising water (Römpp, Lexikon der Chemie, Bleicherde, 10. Auflage, Stuttgart, 1996, page 469). Particularly suitable bleaching earths are attapulgite, bentonite and montmorillonite.

"Acid activated bleaching earths" are bleaching earths which are treated by strong mineral acids. This acid treatment leads to ion exchange in the bleaching earth. Due to this ion-exchange, i.e. acid activation, the properties of the bleaching earths are strongly modified.

The acid activated bleaching earth is preferably an acid activated bentonite. Particularly suitable are acid activated bleaching earths which are commercialized under the trademark TONSIL® by Süd-Chemie.

In a further embodiment the acid activated bleaching earths are those commercialized as activated clays F-20 and F-20X by BASF.

The acid activated bleaching earth has preferably a specific surface area (BET) between 150 and 400 $m^2/g$, preferably between 250 and 375 $m^2/g$, more preferably between 300 and 350 $m^2/g$. The specific surface area is measured according to the method BET (**B**runauer, **E**mmett und **T**eller) which is a commonly known method for determination of surface areas of solid particles by physical adsorption of gas, particularly as described in ISO 9277.

Particularly a 10 % by weight suspension in water has a pH of between 1.5 and 5, preferably between 1.5 and 4, measured at 25°C and after filtration.

The acidity of the acid activated bleaching earth, measured as mg KOH/g, is preferably in the range of 0.1 to 22, more preferably in the range of 0.2 to 21, even more preferably in the range of 0.3 to 20.

Furthermore, it is preferred that the acid activated bleaching earth has after drying at 110°C during 2 hours a residual amount of CaO of less than 1.5 % by weight, particularly between 0.001 and 0.8 % by weight.

Particularly suitable are the products of the trademark TONSIL® SUPREME such as TONSIL® SUPREME 110 FF, TONSIL® SUPREME 112 FF, TONSIL® SUPREME 114 FF, TONSIL® SUPREME 115 FF and TONSIL® SUPREME 118 FF and TONSIL® Optimum 210 FF allowing a fast separation, particularly a fast filtration. Furthermore suitable are the products sold as activated clays F-20 and F-20X by BASF. It has been observed that TONSIL® SUPREME 115 FF, activated clay F-20, and F-20X are particularly suitable for the purpose of the present invention.

Acid activated bleaching earths are very interesting in view of toxicological aspects. Acid activated bleaching earths are particularly suitable for the treatment of food and feed ingredients, since for a long time already, acid activated bleaching earths are used on an industrial scale for the treatment of vegetable oils and fats. The acid activated bleaching earths can also be easily removed completely from the tocopherols.

Tocopherol of formula (I) is a known product as such and is part of vitamin E. It can be produced in different ways as disclosed in Ullmann's Encyclopedia of Industrial Chemistry, Release 2010, 7th Edition, "Vitamins", pages 43 - 47 or in WO 2005/054223 A2.

The preferred route of synthesis is the condensation of the corresponding methyl-, dimethyl- or trimethylhydoquinone of formula (III) with phytol or isophytol.

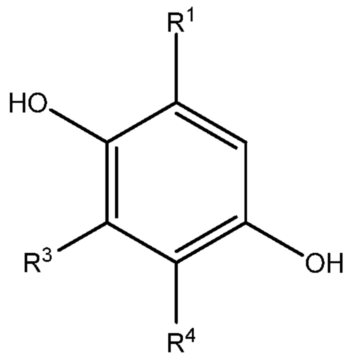

(III)

For this condensation a series of catalysts may be used such as $ZnCl_2$/mineral acid, $BF_3/AlCl_3$, Fe/HCl, trifluoroacetic acid or boric acid/carboxylic acid as well as indium (III) or scandium (III) salts as disclosed in WO 2005/121115 A1. Furthermore suitable catalysts are heteropoly acids, particularly 12-tungstophosphoric or 12-tungstosilicic acid.

Said reactions are not stereospecific and, hence, a mixture of isomers of tocopherol of formula (I) is obtained. This is reflected by the use of the * in the formulae of this document. Due to the fact that there are 3 chiral centres in compounds of formula (I) there exist 8 individual isomers. Such a product being a mixture of R- and S- configuration at each chiral centre (2, 4' and 8') indicated by * in this document is also called (*all-rac*)-tocopherol.

In view of industrial interest and volume of production it is preferred that tocopherol of formula (I) is (*all-rac*)-tocopherol, particularly (*all-rac*)--$\alpha$-tocopherol.

Since the different isomers have different bioactivity it is in the interest to synthesize specifically a desired isomer having high bioactivity. (2R, 4'R, 8'R)-tocopherol isomers have shown to exhibit a particularly high bioactivity. There exist such stereoselective syntheses which yield to either specific isomers or mixtures with less isomers only. For example, natural phytol can be used for the above synthesis. Natural phytol consists only of the R,R-isomer and, hence, is isomerically pure. Therefore, the above reaction leads to a mixture of (2R, 4'R, 8'R)-tocopherol and (2S, 4'R, 8'R)-tocopherol, also known as 2-*ambo*-tocopherol. However, as natural phytol, or isophytol, is commercially available only in rather small amounts and is rather expensive, the potential of using natural phytol, or isophytol, for industrial scale synthesis of tocopherols is rather limited.

Is has been found, however, that by using specific chiral iridium complexes, asymmetrical hydrogenation of alkenes can be achieved. The chiral iridium complexes disclosed in WO 2006/066863 A1 as well as in the pending application EP11169784.3 are particularly suited for this purpose. By this use of asymmetrical hydrogenation 2-*ambo*-tocopherol can be obtained, the isomers of which can be separated much more easily, more efficiently and particularly in a much more cost effective way than isomeric mixtures obtained by traditional processes.

Therefore, it is preferred in another embodiment that the tocopherol of formula (I) is a mixture of (2R, 4'R, 8'R)-tocopherol and (2S, 4'R, 8'R)-tocopherol.

More preferred, the tocopherol of formula (I) is (2R, 4'R, 8'R)-tocopherol.

Most preferred, the tocopherol of formula (I) is (2R, 4'R, 8'R)-$\alpha$-tocopherol.

Preferred are the following combinations of $R^1$, $R^3$ and $R^4$:

$R^1 = R^3 = R^4 = CH_3$
or
$R^1 = R^4 = CH_3$, $R^3 = H$
or
$R^1 = H$, $R^3 = R^4 = CH_3$
or
$R^1 = R^3 = H$, $R^4 = CH_3$

Most preferred is the combination $R^1 = R^3 = R^4 = CH_3$.

Therefore, preferably the tocopherol of formula (I) is selected from the group consisting of α-tocopherol, β-tocopherol, γ-tocopherol, δ-tocopherol, particularly α-tocopherol.

It is preferred that only one type of tocopherol of formula (I), is present when the contacting with the acid activated bleaching earth is performed. In other words, it is preferred that no mixtures of different tocopherols with different groups $R^1$, $R^3$ and $R^4$, particularly no mixtures comprising α-tocopherol and γ-tocopherol, are contacted with acid activated bleaching earth.

A key element of the present invention is the interaction of acid activated bleaching earth with the tocopherol of formula (I), in the form of neat or in form of highly concentrated solution tocopherol. This interaction requires a physical contact of the tocopherol with the acid activated bleaching earth. It is desired that the contact is thorough. Hence, any possibility allowing a thorough contact between the surface of the acid activated bleaching earth with the tocopherol of formula (I) can be principally used.

A preferred possibility for such a thorough contact, which is particularly suitable for the purpose of the invention, is when the contacting is realized by adding the acid activated bleaching earth to the tocopherol of formula (I) and stirring. This leads to a suspension of acid activated bleaching earth in a liquid phase comprising the tocopherol of formula (I). This can be realized in a continuous or in a batchwise manner in a stirred vessel or loop reactor or a cascade of those reactor types.

There exist also other possibilities of contacting. The contact with the acid activated bleaching earth can be realized for example by a flow of the tocopherol over the acid activated bleaching earth being immobilized on a carrier such as a plate or a wall or by a flow through a column filled with acid activated bleaching earth. In order to increase the time of contact between activated bleaching earth and the tocopherol it is advisable to use a flow in a continuous loop. However, it has been observed that particularly the use of a fixed bed reactor is disadvantageous as the contact is not sufficient enough to allow an industrially effective process at an acceptable pressure drop.

It is preferred that the contacting is performed under inert conditions, particularly under nitrogen.

Furthermore, it has been shown that the contact is particularly efficient when the contacting is done at elevated temperature, particularly at a temperature of between 80 and 160 °C, preferably between 100 and 150°C.

It is further preferred that the time of contact is between 30 and 300 minutes, preferably between 45 and 180 minutes, more preferably between 60 and 120 minutes.

Particularly preferred is when the contact is realized at an absolute pressure of 0.01 - 1013 mbar, preferably 10 - 500 mbar, more preferably 20 - 200 mbar.

The step of contacting can be realized in a continuous or in a batch process.

It has been observed that it is preferred that the weight ratio of acid activated bleaching earth to tocopherol of formula (I) is between 0.5 / 100 and 5 / 100, particularly is between 1 / 100 and 3 / 100, preferably between 1 / 100 and 2 / 100. For example, using 5 g acid activated bleaching earth per 100 g tocopherol of formula (I) leads to a weight ratio of acid activated bleaching earth to tocopherol of formula (I) of 5 / 100.

The tocopherol of formula (I) is contacted as such, i.e. in the form of neat tocopherol of formula (I), or in the form of a solution comprising more than 60 %, preferably more than 90 %, particularly more than 95 %, with the acid activated bleaching earth. For the solution a non-polar solvent, particularly selected from the group consisting of hexane, heptane, xylene and toluene is preferred.

The use of solvents to yield a solution is advantageous in view of lowering the viscosity of the tocopherol of formula (I). A lower viscosity enables a better contact, particularly at lower temperatures and an easier and faster separation from the acid activated bleaching earth.

As the method of invention focuses particularly to tocopherols originating from chemical synthetic processes, any solution should also not comprise any triglycerides or any fatty acids, particularly as they occur in vegetable oils.

After contacting of the tocopherol of formula (I) with an acid activated bleaching earth the acid activated bleaching earth is separated from the tocopherol of formula (I) in a further step. The separation can be achieved for example by filtering off the acid activated bleaching earth after the contacting or by centrifugation.

Color stability

It has been observed that the method of this invention leads to an increased color stability of the tocopherol of formula (I).

Colors are difficult to describe. One of the methods is the representation of color by the so-called *Lab color space.* Currently the mostly used Lab method is the CIE-$L^*a^*b^*$ as proposed by CIE in 1976. In this representation the parameters "L", resp. "$L^*$", is lightness, and the parameters "a", resp. "$a^*$", and "b", resp. "$b^*$", are color-opponent dimensions. The parameter L, resp. $L^*$, has a value between 0 and 100, parameters a and b, resp. $a^*$ and $b^*$, have a value between -100 and 100.

Changes in color are, hence, defined by a change of L, resp. $L^*$, (= ΔL resp. $\Delta L^*$), a, resp. $a^*$, (= Δa resp. $\Delta a^*$),

and/or b, resp. $b^*$, (= $\Delta b$ resp. $\Delta b^*$) values.

Despite the background theory is rather complex, a decrease of value of L* leads to a sensation of a darker color. An increase of the value a* leads to a sensation of enhancing of the red color component. An increase of the value b* leads to a sensation of enhancing of the yellow color component.

A color change is usually not only a change of an individual parameter L, a or b, resp. $L^*$, $a^*$ or $b^*$, but a change of more than one of this parameters simultaneously.

Hence, there has been the color difference defined which is expressed by $\Delta E$, resp. $\Delta E^*$, according to the following formula

$$\Delta E = \sqrt{\Delta L^2 + \Delta a^2 + \Delta b^2}$$

$$\Delta E* = \sqrt{\Delta L*^2 + \Delta a*^2 + \Delta b*^2}$$

Assessing the advantage of this invention is best realized when the color and color stability of the tocopherol of formula (I) prepared by the method of invention (*"inventive tocopherol*") is compared with the corresponding tocopherol ("*comparative tocopherol*") which has been identically prepared except that it has not been contacted with an acid activated bleaching earth.

It is important to realize that the <u>initial</u> color of the *inventive tocopherol* is almost identical to the one of the *comparative tocopherol.*

However, the *inventive tocopherol* exhibits a much pronounced color <u>stability</u> compared with the *comparative tocopherol.*

The *inventive tocopherol* is directly after preparation nearly colorless. The value L* is more than 95, and value of a* is between 0 and -5, and the value of b* is between 0 and less than 15.

The degree of color stability in the context of this invention is defined in view of the degree of change of the parameters L, a or b, resp. $L^*$, $a^*$ or $b^*$, i.e. by $\Delta L^*$, $\Delta a^*$ or $\Delta b^*$, resp. $\Delta L$, $\Delta a$ or $\Delta b$, over time of storage always relative to the initial values, i.e. before storage. More specifically it is defined by the color difference $\Delta E^*$ over time of storage.

On the one hand, it has been particularly found that the *inventive tocopherol* shows a much lower decrease of the value L, resp. $L^*$, than the *comparative tocopherol.* It has been found, particularly when the measurements are made in glass cuvettes of diameters 10 mm, as described in more detail in the experimental part, that the ratio of the $\Delta L^*$ of the *inventive tocopherol* to the $\Delta L^*$ of the *comparative tocopherol* is typically less than 0.7, particularly less than 0.6, after 10 days of storage at 25°C in an open flask (exposure to air). After 30 days storage under these conditions, this ratio is even less than 0.5, particularly less than 0.4. The value of $L^*$ of the *inventive tocopherol* is preferably larger than 85 after 10 days and larger than 80, preferably larger than 85, after 30 days of storage at 25°C in an open flask (exposure to air).

On the other hand, it has been particularly found that the *inventive tocopherol* shows only a very small change in the parameter a*, whereas the *comparative tocopherol* shows typically a very strong increase of the parameter a*. It has been found, particularly when the measurements are made in glass cuvettes of diameters 10 mm, as described in more detail in the experimental part, that the ratio of the $\Delta a^*$ of the *inventive tocopherol* to the $\Delta a^*$ of the *comparative tocopherol* is typically less than 0.25, particularly less than 0.20, after 10 days of storage at 25°C in an open flask (exposure to air). After 30 days storage under these conditions, this ratio is even less than 0.15, particularly less than 0.10. The value of $a^*$ of the *inventive tocopherol* is preferably between 0 and 2 after 10 days and between 0 and 3, preferably between 0 and 2, after 30 days of storage at 25°C in an open flask (exposure to air).

On the other hand, it has been particularly found that the *inventive tocopherol* shows a much lower decrease of the value b, resp. $b^*$, than the *comparative tocopherol.* It has been found, particularly when the measurements are made in glass cuvettes of diameters 10 mm, as described in more detail in the experimental part, that the ratio of the $\Delta b^*$ of the *inventive tocopherol* to the $\Delta b^*$ of the *comparative tocopherol* is typically less than 0.9, particularly less than 0.8, after 10 days of storage at 25°C in an open flask (exposure to air). After 30 days storage under these conditions, this ratio is even less than 0.9, particularly less than 0.8. The value of $b^*$ of the *inventive tocopherol* is preferably between 0 and 40, preferably between 0 and 30, after 10 days and between 0 and 50, preferably between 0 and 45, after 30 days of storage at 25°C in an open flask (exposure to air).

On the other hand, it has been particularly found that the *inventive tocopherol* shows a much lower color difference $\Delta E$, resp. $\Delta E^*$, than the *comparative tocopherol.* It has been found, particularly when the measurements are made in glass cuvettes of diameters 10 mm, as described in more detail in the experimental part, that the ratio of the $\Delta E^*$ of the *inventive tocopherol* to the $\Delta E^*$ of the *comparative tocopherol* is typically less than 0.8, particularly less than 0.7, after 10 days of storage at 25°C in an open flask (exposure to air). After 30 days storage under these conditions,

this ratio is even less than 0.7, particularly less than 0.65. The value of $\Delta E^*$ of the *inventive tocopherol* is preferably between 0 and 30, preferably between 0 and 25, after 10 days and between 0 and 50, preferably between 0 and 40, after 30 days of storage at 25°C in an open flask (exposure to air).

The method mentioned before is characterized in that the increased color stability is defined by showing a lower $\Delta E^*_t$ than the corresponding tocopherol of formula (I), which has not been contacted with an acid activated bleaching earth

$$\Delta E^*_t = \sqrt{(L^*_o - L^*_t)^2 + (a^*_0 - a^*_t)^2 + (b^*_0 - b^*_t)^2}$$

The $L^*_0$, resp. $a^*_0$, resp. $b^*_0$ being the L* value, resp. a* value, resp. b* value measured before the storage and $L^*_t$, resp. $a^*_t$, resp. $b^*_t$ being the L* value, resp. a* value, resp. b* value measured after a specific time (t) of storage and exposure to air which is at least 200 hours at 25°C. The L* values and a* values and b* values are measured according to the CIE-L*a*b* method.

The value of $\Delta E^*_{237\ hours}$, respectively as discussed above the value of $\Delta E^*_{10\ days}$, for the *inventive tocopherol,* i.e. the tocopherol of formula (I) being prepared according to the method of invention, is between 0 and 30, preferably between 0 and 25 (i.e. after 237 hours, resp. 10 days, of storage at 25°C and exposure to air). The value of $\Delta E^*_{722\ hours}$, respectively as discussed above the value of $\Delta E^*_{30\ days}$, for the *inventive tocopherol,* i.e. the tocopherol of formula (I) being prepared according to the method of invention, is between 0 and 50, preferably between 0 and 40, after 722 hours, resp. 30 days of storage at 25°C in an open flask (exposure to air).

Preferably, the method further comprises the subsequent steps

a) measuring the L* value and a* value and b* value of the tocopherol of formula (I) which has been contacted with the acid activated bleaching earth before and after a specific time (t) of storage and exposure to air which is at least 200 hours at 25°C and calculating thereof the value $\Delta E^*_t$

$$\Delta E^*_t = \sqrt{(L^*_o - L^*_t)^2 + (a^*_0 - a^*_t)^2 + (b^*_0 - b^*_t)^2}$$

$L^*_0$, resp. $a^*_0$, resp. $b^*_0$ being the L* value, resp. a* value, resp. b* value measured before the storage and $L^*_t$, resp. $a^*_t$, resp. $b^*_t$ being the L value, resp. a value, resp. b value measured after a specific time (t) of storage and exposure;

b) measuring the L* value and a* value and b* value of the corresponding tocopherol of formula (I) which has <u>not</u> been contacted with an acid activated bleaching earth before and after the specific time (t) of storage and exposure to air selected in step a) and calculating thereof the value $\Delta E^*_t$

$$\Delta E^*_t = \sqrt{(L^*_o - L^*_t)^2 + (a^*_0 - a^*_t)^2 + (b^*_0 - b^*_t)^2}$$

$L^*_0$, resp. $a^*_0$, resp. $b^*_0$ being the L* value, resp. a* value, resp. b* value measured before the storage and $L^*_t$, resp. $a^*_t$, resp. $b^*_t$ being the L* value, resp. a* value, resp. b* value measured after a specific time (t) of storage and exposure;

c) comparing the value $\Delta E^*_t$ measured in step a) with the value $\Delta E^*_t$ measured in step b);

d) detecting that the value $\Delta E^*_t$ measured in step a) is lower than the value $\Delta E^*_t$ measured in step b);

wherein the L* values and a* values and b* values are measured according to the CIE-L*a*b* method.

Finally, it is important to stress that this method does not need any additive which needs to remain continuously in the tocopherol of formula (I) which eventually would restrict the possible fields of applications of the tocopherols because of toxicological doubts or limitations of such an additive. The current method, however, has no such limitations and is therefore optimal for using tocopherols in pharma, food or feed applications.

In a further aspect the invention relates to the use of an acid activated bleaching earth for the discoloration of tocopherol of formula (I), in the form of neat tocopherol of formula (I), or in the form of a solution comprising more than 60 % by weight of tocopherol of formula (I). The tocopherol of formula (I) has been already described above in detail.

The tocopherol of formula (I) can be used in different fields. Preferably it can be used in the field of pharma, food or feed.

Therefore, particularly the process of preparing a food or feed composition with an increased color stability comprising the steps of

i) preparing a tocopherol of formula (I) with an increased color stability according to the method as described above; and

ii) adding tocopherol of formula (I) with an increased color stability yielding from step i) to at least one dietary supplement or food or feed ingredient;

represents a further aspect of the present inventions.

## Examples

Comparative example ***Ref.1***:

100 kg (*all-rac*)-α-tocopherol was added to the reactor. In this reactor the sample was stirred at an absolute pressure of 50-60 mbar under nitrogen during 90 minutes at 130 °C. Afterwards the sample was rectified under vacuum by a rectification column.

Example ***1***

As in comparative example ***Ref. 1,*** 100 kg (*all-rac*)-α-tocopherol was added to the reactor. In this reactor 2 kg of TONSIL® SUPREME 115 FF has been added and stirred at an absolute pressure of 50-60 mbar under nitrogen during 90 minutes at 130 °C. Afterwards the acid activated bleaching earth was filtered off. Then the sample was rectified under identical conditions as used for comparative example ***Ref. 1.*** Except the contacting with the acid activated bleaching earth, the example ***1,*** hence, has been prepared identically as the comparative example ***Ref.1.***

Storage samples ***Ref.1*** and ***1*** & Results:

Samples of ***Ref.1*** and ***1*** have been taken and filled into glass cuvettes with 10 mm diameter and 8.5 cm length, so that each cuvette showed a filling level of 4.5 cm (measured from the button) with the substance to be tested. The cuvettes were not closed.

For the measurement a sample (cuvette) was taken, stirred and the values $L^*$, $a^*$ and $b^*$ have been measured on a LICO 200 (Dr. Lange) according to the CIE-$L^*a^*b^*$ method. These measured values are represented in table 1 as $L^*_0$, resp. $a^*_0$, resp. $b^*_0$.

The other cuvettes of example ***1,*** resp. ***Ref.1***, have been stored at 25°C at exposure to air (by not closing the cuvettes). After the time *t* indicated in table 1 a cuvette was taken, stirred and the values of $L^*_t$, resp. $a^*_t$, resp. $b^*_t$, have been measured accordingly. The values measured are represented in table 1.

The color difference $\Delta E^*_t$ was calculated from the corresponding values using the formula

$$\Delta E^*_t = \sqrt{(L^*_o - L^*_t)^2 + (a^*_0 - a^*_t)^2 + (b^*_0 - b^*_t)^2}$$

and is represented in table 1.

Table 1 Color change of ***Ref.1*** and ***1*** measured by CIE-$L^*a^*b^*$ method.

| | ***Ref.1*** | | | | ***1*** | | | |
|---|---|---|---|---|---|---|---|---|
| Time of storage *t* [h] | $L^*_t$ | $a^*_t$ | $b^*_t$ | $\Delta E^*_t$ | $L^*_t$ | $a^*_t$ | $b^*_t$ | $\Delta E^*_t$ |
| 0 | 97.8 | -1.9 | 10.3 | 0.0 | 99.1 | -1.6 | 6.8 | 0.0 |
| 6 | 96.9 | -1.3 | 11.6 | 1.7 | 98.2 | -1.2 | 8.0 | 1.6 |
| 22 | 95.2 | -0.4 | 15.7 | 6.2 | 98.2 | -1.2 | 8.4 | 1.9 |
| 45 | 94.7 | -0.2 | 16.4 | 7.1 | 97.5 | -1.0 | 9.9 | 3.5 |
| 141 | 91.3 | 1.8 | 22.9 | 14.7 | 94.1 | -0.6 | 18.5 | 12.8 |
| 189 | 86.0 | 5.2 | 32.9 | 26.5 | 92.9 | -0.3 | 21.5 | 16.0 |
| 213 | 86.4 | 5.2 | 32.1 | 25.6 | 92.9 | -0.4 | 22.9 | 17.3 |
| 237 | 83.7 | 7.0 | 37.9 | 32.2 | 91.0 | -0.1 | 26.5 | 21.4 |
| 309 | 81.2 | 9.2 | 41.8 | 37.3 | 89.7 | 0.7 | 33.1 | 28.0 |
| 381 | 79.5 | 10.8 | 42.2 | 38.9 | 88.1 | 1.3 | 36.2 | 31.5 |

(continued)

| | *Ref.1* | | | | *1* | | | |
|---|---|---|---|---|---|---|---|---|
| Time of storage *t* [h] | $L^*_t$ | $a^*_t$ | $b^*_t$ | $\Delta E^*_t$ | $L^*_t$ | $a^*_t$ | $b^*_t$ | $\Delta E^*_t$ |
| 552 | 66.9 | 23.0 | 61.0 | 64.4 | 87.3 | 1.4 | 41.1 | 36.4 |
| 722 | 66.4 | 23.6 | 61.1 | 64.9 | 87.4 | 0.7 | 44.2 | 39.3 |
| 912 | 63.0 | 27.1 | 64.9 | 70.9 | 86.2 | 1.0 | 50.5 | 45.6 |

One can clearly derive from table 1 that the example ***1*** exhibits a much better color stability than the corresponding comparison example ***Ref.1***.

Figure 1 is a graphical representation of the values $L^*_t$ of example ***1*** (circles, dashed smoothed line) and of comparison example ***Ref.1*** (triangles, straight smoothed line).

Figure 2 is a graphical representation of the values $a^*_t$ of example ***1*** (circles, dashed smoothed line) and of comparison example ***Ref.1*** (triangles, straight smoothed line).

Figure 3 is a graphical representation of the values $b^*_t$ of example ***1*** (circles, dashed smoothed line) and of comparison example ***Ref.1*** (triangles, straight smoothed line).

Figure 4 is a graphical representation of the values $\Delta E^*_t$ of example ***1*** (circles, dashed smoothed line) and of comparison example ***Ref.1*** (triangles, straight smoothed line).

## Claims

**1.** Method of preparing tocopherol of formula (I) with an increased color stability comprising the step of contacting tocopherol of formula (I)), in the form of neat tocopherol of formula (I) or in the form of a solution comprising more than 60 % by weight of tocopherol of formula (I), with an acid activated bleaching earth.

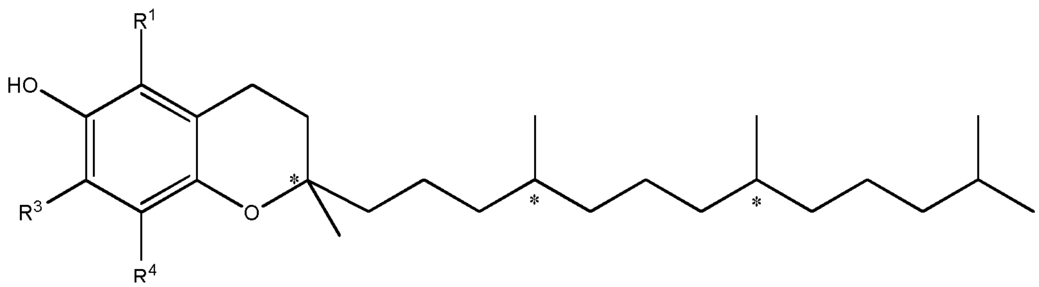

(I)

wherein $R^1$, $R^3$ and $R^4$ are independently from each other hydrogen or methyl groups;
and wherein each * represents an individual chiral centre;

**characterized in that**
the increased color stability is defined by showing a lower $\Delta E^*_t$ than the corresponding tocopherol of formula (I), which has not been contacted with an acid activated bleaching earth
wherein

$$\Delta E^*_t = \sqrt{(L^*_o - L^*_t)^2 + (a^*_0 - a^*_t)^2 + (b^*_0 - b^*_t)^2}$$

$L^*_0$, resp. $a^*_0$, resp. $b^*_0$ being the L* value, resp. a* value, resp. b* value measured before the storage and

$L^*_t$, resp. $a^*_t$, resp. $b^*_t$ being the L* value, resp. a* value, resp. b* value measured after a specific time (t) of

storage and exposure to air which is at least 200 hours at 25°C;

wherein the $L^*$ values and $a^*$ values and $b^*$ values are measured according to the CIE-$L^*a^*b^*$ method.

**2.** Method according to claim 1 **characterized in that** the acid activated bleaching earth is an acid activated bentonite.

**3.** Method according to anyone of the preceding claims **characterized in that** the acid activated bleaching earth has a specific surface area (BET) between 150 and 400 $m^2/g$, preferably between 250 and 375 $m^2/g$, more preferably between 300 and 350 $m^2/g$.

**4.** Method according to anyone of the preceding claims **characterized in that** tocopherol of formula (I) is $\alpha$-tocopherol.

**5.** Method according to anyone of the preceding claims **characterized in that** the contacting is done at a temperature of between 80 and 160 °C, preferably between 100 and 150°C.

**6.** Method according to anyone of the preceding claims **characterized in that** the contacting is realized **in that** the acid activated bleaching earth is added to the tocopherol of formula (I) and stirred.

**7.** Method according to anyone of the preceding claims **characterized in that** weight ratio of acid activated bleaching earth to tocopherol of formula (I) is between 0.5 / 100 and 5 / 100, particularly is between 1 / 100 and 3 / 100, preferably between 1 / 100 and 2 / 100.

**8.** Method according to anyone of the preceding claims **characterized in that** the tocopherol in the form of neat tocopherol of formula (I) does not comprise any triglycerides or any fatty acids.

**9.** Method according to anyone of the preceding claims **characterized in that** it further comprises the subsequent steps

a) measuring the $L^*$ value and $a^*$ value and resp. $b^*$ value of the tocopherol of formula (I) which has been contacted with the acid activated bleaching earth before and after a specific time (t) of storage and exposure to air which is at least 200 hours at 25°C and calculating thereof the value $\Delta E^*_t$

$$\Delta E^*_t = \sqrt{(L^*_o - L^*_t)^2 + (a^*_0 - a^*_t)^2 + (b^*_0 - b^*_t)^2}$$

$L^*_0$, resp. $a^*_0$, resp. $b^*_0$ being the $L^*$ value, resp. $a^*$ value, resp. $b^*$ value measured before the storage and $L^*_t$, resp. $a^*_t$, resp. $b^*_t$ being the L value, resp. a value, resp. b value measured after a specific time (t) of storage and exposure;

b) measuring the $L^*$ value and $a^*$ value and $b^*$ value of the corresponding tocopherol of formula (I) which has not been contacted with an acid activated bleaching earth before and after the specific time (t) of storage and exposure to air selected in step a) and calculating thereof the value $\Delta E^*_t$

$$\Delta E^*_t = \sqrt{(L^*_o - L^*_t)^2 + (a^*_0 - a^*_t)^2 + (b^*_0 - b^*_t)^2}$$

$L^*_0$, resp. $a^*_0$, resp. $b^*_0$ being the $L^*$ value, resp. $a^*$ value, resp. $b^*$ value measured before the storage and $L^*_t$, resp. $a^*_t$, resp. $b^*_t$ being the $L^*$ value, resp. $a^*$ value, resp. $b^*$ value measured after a specific time (t) of storage and exposure;

c) comparing the value $\Delta E^*_t$ measured in step a) with the value $\Delta E^*_t$ measured in step b);

d) detecting that the value $\Delta E^*_t$ measured in step a) is lower than the value $\Delta E^*_t$ measured in step b);

wherein the $L^*$ values and $a^*$ values and $b^*$ values are measured according to the CIE-$L^*a^*b^*$ method.

**10.** Use of an acid activated bleaching earth for the discoloration of tocopherol of formula (I), in the form of neat tocopherol of formula (I), or in the form of a solution comprising more than 60 % by weight of tocopherol of formula (I),

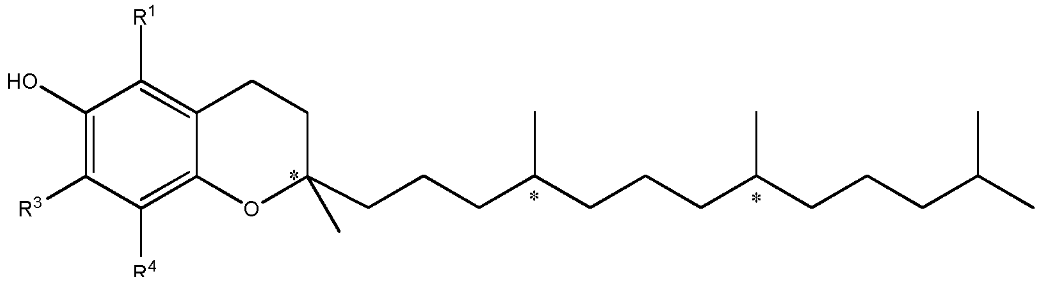

(I)

wherein $R^1$, $R^3$ and $R^4$ are independently from each other hydrogen or methyl groups; and wherein each * represents an individual chiral centre.

**11.** Process of preparing a food or feed composition with an increased color stability comprising the steps of

i) preparing a tocopherol of formula (I) with an increased color stability according to the method of claims 1 - 9; and
ii) adding tocopherol of formula (I) with an increased color stability yielding from step i) to at least one dietary supplement or food or feed ingredient;

**Patentansprüche**

**1.** Verfahren zur Herstellung von Tocopherol der Formel (I) mit erhöhter Farbstabilität, umfassend den Schritt des Inkontaktbringens von Tocopherol der Formel (I), in Form von Tocopherol der Formel (I) in Substanz oder in Form einer Lösung umfassend mehr als 60 Gew.-% Tocopherol der Formel (I), mit einer säureaktivierten Bleicherde,

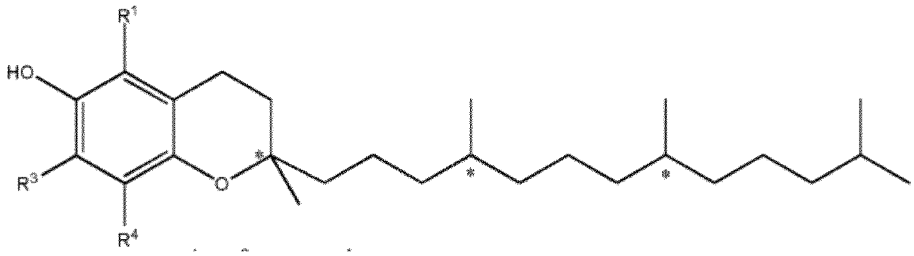

(I)

wobei $R^1$, $R^3$ und $R^4$ unabhängig voneinander Wasserstoff oder Methylgruppen bedeuten; und wobei jedes * ein einzelnes Chiralitätszentrum darstellt;

**dadurch gekennzeichnet, dass**
die erhöhte Farbstabilität durch einen niedrigeren $\Delta E^*_t$-Wert als das entsprechende Tocopherol der Formel (I), das nicht mit einer säureaktivierten Bleicherde in Kontakt gebracht worden ist, definiert ist,
wobei

$$\Delta E^*_t = \sqrt{(L^*_o - L^*_t)^2 + (a^*_0 - a^*_t)^2 + (b^*_0 - b^*_t)^2}$$

$L^*_0$ bzw. $a^*_0$ *bzw.* $b^*_0$ der vor der Lagerung gemessene $L^*$-Wert bzw. $a^*$-Wert bzw. $b^*$-Wert ist und

$L^*_t$ bzw. $a^*_t$ *bzw.* $b^*_t$ der nach einer spezifischen Zeit (t) der Lagerung und Exposition gegenüber Luft, die mindestens 200 Stunden bei 25°C beträgt, gemessene L*-Wert bzw. a*-Wert bzw. b*-Wert ist;

wobei die L*-Werte und a*-Werte und b*-Werte gemäß der CIE-L*a*b*-Methode gemessen werden.

**2.** Verfahren nach Anspruch 1, **dadurch gekennzeichnet, dass** es sich bei der säureaktivierten Bleicherde um einen

säureaktivierten Bentonit handelt.

3. Verfahren nach einem der vorhergehenden Ansprüche, **dadurch gekennzeichnet, dass** die säureaktivierte Bleicherde eine spezifische Oberfläche (BET) zwischen 150 und 400 $m^2/g$, vorzugsweise zwischen 250 und 375 $m^2/g$, stärker bevorzugt zwischen 300 und 350 $m^2/g$, aufweist.

4. Verfahren nach einem der vorhergehenden Ansprüche, **dadurch gekennzeichnet, dass** es sich bei dem Tocopherol der Formel (I) um $\alpha$-Tocopherol handelt.

5. Verfahren nach einem der vorhergehenden Ansprüche, **dadurch gekennzeichnet, dass** das Inkontaktbringen bei einer Temperatur zwischen 80 und 160°C, vorzugsweise zwischen 100 und 150°C, erfolgt.

6. Verfahren nach einem der vorhergehenden Ansprüche, **dadurch gekennzeichnet, dass** das Inkontaktbringen durch Zusetzen der säureaktivierten Bleicherde zu dem Tocopherol der Formel (I) und Rühren erfolgt.

7. Verfahren nach einem der vorhergehenden Ansprüche, **dadurch gekennzeichnet, dass** das Gewichtsverhältnis von säureaktivierter Bleicherde zu Tocopherol der Formel (I) zwischen 0,5/100 und 5/100, insbesondere zwischen 1/100 und 3/100, bevorzugt zwischen 1/100 und 2/100, liegt.

8. Verfahren nach einem der vorhergehenden Ansprüche, **dadurch gekennzeichnet, dass** das Tocopherol in Form von Tocopherol der Formel (I) in Substanz keine Triglyceride bzw. keine Fettsäuren umfasst.

9. Verfahren nach einem der vorhergehenden Ansprüche, **dadurch gekennzeichnet, dass** es weiterhin die folgenden Schritte umfasst:

a) Messen des L*-Werts und a*-Werts und bzw. b*-Werts des Tocopherols der Formel (I), das mit der säureaktivierten Bleicherde in Kontakt gebracht worden ist, vor und nach einer spezifischen Zeit (t) der Lagerung und Exposition gegenüber Luft, die mindestens 200 Stunden bei 25°C beträgt, und aufgrund dessen Berechnen des Werts $\Delta E^*_t$

$$\Delta E^*_t = \sqrt{(L^*_o - L^*_t)^2 + (a^*_0 - a^*_t)^2 + (b^*_0 - b^*_t)^2} \quad ,$$

wobei $L^*_0$ bzw. $a^*_0$ *bzw.* $b^*_0$ der vor der Lagerung gemessene L*-Wert bzw. a*-Wert bzw. b*-Wert ist und $L^*_t$ bzw. $a^*_t$ *bzw.* $b^*_t$ der nach einer spezifischen Lagerungszeit (t) und Exposition gemessene L-Wert bzw. a-Wert bzw. b-Wert ist;

b) Messen des L*-Werts und a*-Werts und b*-Werts des entsprechenden Tocopherols der Formel (I), das nicht mit einer säureaktivierten Bleicherde in Kontakt gebracht worden ist, vor und nach der in Schritt a) ausgewählten spezifischen Zeit (t) der Lagerung und Exposition gegenüber Luft, und aufgrund dessen Berechnen des Werts $\Delta E^*_t$

$$\Delta E^*_t = \sqrt{(L^*_o - L^*_t)^2 + (a^*_0 - a^*_t)^2 + (b^*_0 - b^*_t)^2} \quad ,$$

wobei $L^*_0$ bzw. $a^*_0$ *bzw.* $b^*_0$ der vor der Lagerung gemessene L*-Wert bzw. a*-Wert bzw. b*-Wert ist und $L^*_t$ bzw. $a^*_t$ *bzw.* $b^*_t$ der nach einer spezifischen Zeit (t) der Lagerung und Exposition gemessene L*-Wert bzw. a*-Wert bzw. b*-Wert ist;

c) Vergleichen des in Schritt a) gemessenen $\Delta E^*_t$-Werts mit dem in Schritt b) gemessenen $\Delta E^*_t$-Wert;

d) Detektieren, dass der in Schritt a) gemessene $\Delta E^*_t$-Wert niedriger als der in Schritt b) gemessene $\Delta E^*_t$-Wert ist;

wobei die L*-Werte und a*-Werte und b*-Werte gemäß der CIE-L*a*b*-Methode gemessen werden.

10. Verwendung einer säureaktivierten Bleicherde zum Entfärben von Tocopherol der Formel (I), in Form von Tocopherol der Formel (I) in Substanz oder in Form einer Lösung umfassend mehr als 60 Gew.-% Tocopherol der Formel (I)

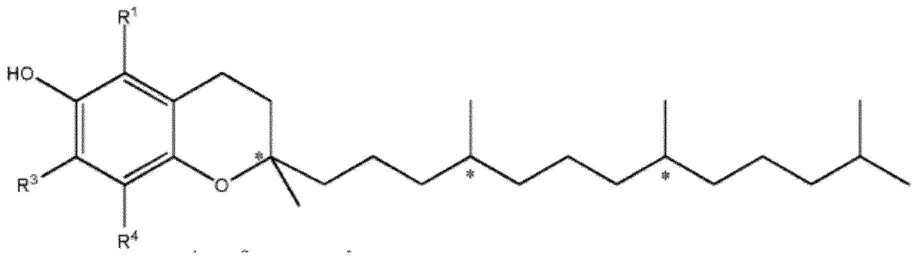

(I)

wobei $R^1$, $R^3$ und $R^4$ unabhängig voneinander Wasserstoff oder Methylgruppen bedeuten; und wobei jedes * ein einzelnes Chiralitätszentrum darstellt.

**11.** Verfahren zur Herstellung einer Nahrungs- oder Futtermittelzusammensetzung mit erhöhter Farbstabilität, umfassend die folgenden Schritte:

i) Herstellen eines Tocopherols der Formel (I) mit erhöhter Farbstabilität nach dem Verfahren der Ansprüche 1-9; und

ii) Zusetzen des in Schritt i) erhaltenen Tocopherols der Formel (I) mit erhöhter Farbstabilität zu mindestens einem Nahrungsergänzungsmittel oder Nahrungsmittel- oder Futterbestandteil;

## Revendications

**1.** Procédé de préparation de tocophérol de formule (I) avec une stabilité de couleur accrue comprenant l'étape de mise en contact du tocophérol de formule (I), sous la forme de tocophérol de formule (I) pur ou sous la forme d'une solution comprenant plus de 60 % en poids de tocophérol de formule (I), avec une terre de décolorante activée par acide

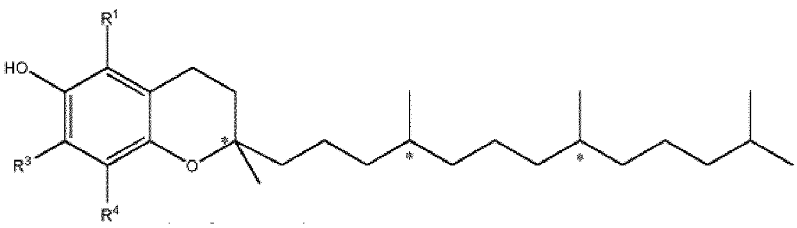

(I)

dans lequel $R^1$, $R^3$ et $R^4$ représentent indépendamment les uns des autres un hydrogène ou un groupe méthyle ; et dans lequel chaque * représente un centre chiral individuel ;

**caractérisé en ce que**
la stabilité de couleur accrue est définie comme le fait de présenter un $\Delta E^*_t$ inférieur à celui du tocophérol de formule (I) correspondant qui n'a pas été mis en contact avec une terre décolorante activée par acide dans lequel

$$\Delta E^*_t = \sqrt{(L^*_0 - L^*_t)^2 + (a^*_0 - a^*_t)^2 + (b^*_0 - b^*_t)^2}$$

$L^*_0$, respectivement $a^*_0$, respectivement $b^*_0$ étant la valeur de L*, respectivement la valeur de a*, respectivement la valeur de b* mesurée avant le stockage et
$L^*_t$, respectivement $a^*_t$, respectivement $b^*_t$ étant la valeur de L*, respectivement la valeur de a*, respectivement la valeur de b* mesurée après un temps spécifique (t) de stockage et d'exposition à l'air qui est d'au moins 200 heures à 25 °C ;
dans lequel les valeurs de L* et les valeurs de a* et les valeurs de b* sont mesurées selon la méthode CIE-L*a*b*.

**2.** Procédé selon la revendication 1 **caractérisé en ce que** la terre décolorante activée par acide est une bentonite

activée par acide.

3. Procédé selon l'une quelconque des revendications précédentes **caractérisé en ce que** la terre décolorante activée par acide a une surface spécifique (BET) comprise entre 150 et 400 $m^2/g$, de préférence entre 250 et 375 $m^2/g$, mieux encore entre 300 et 350 $m^2/g$.

4. Procédé selon l'une quelconque des revendications précédentes **caractérisé en ce que** le tocophérol de formule (I) est l'$\alpha$-tocophérol.

5. Procédé selon l'une quelconque des revendications précédentes **caractérisé en ce que** la mise en contact est effectuée à une température comprise entre 80 et 160 °C, de préférence entre 100 et 150 °C.

6. Procédé selon l'une quelconque des revendications précédentes **caractérisé en ce que** la mise en contact est réalisée de telle sorte que la terre décolorante activée par acide est ajoutée au tocophérol de formule (I) et agitée.

7. Procédé selon l'une quelconque des revendications précédentes **caractérisé en ce que** le rapport pondéral entre la terre décolorante activée par acide et le tocophérol de formule (I) se situe entre 0,5/100 et 5/100, en particulier se situe entre 1/100 et 3/100, de préférence entre 1/100 et 2/100.

8. Procédé selon l'une quelconque des revendications précédentes **caractérisé en ce que** le tocophérol sous la forme de tocophérol de formule (I) pur ne comprend aucun triglycéride ni aucun acide gras.

9. Procédé selon l'une quelconque des revendications précédentes **caractérisé en ce qu'**il comprend en outre les étapes suivantes :

a) mesurer la valeur de L* et la valeur de a* et respectivement la valeur de b* du tocophérol de formule (I) qui a été mis en contact avec la terre décolorante activée par acide avant et après un temps spécifique (t) de stockage et d'exposition à l'air qui est d'au moins 200 heures à 25 °C et en calculer la valeur $\Delta E^*_t$

$$\Delta E^*_t = \sqrt{(L^*_0 - L^*_t)^2 + (a^*_0 - a^*_t)^2 + (b^*_0 - b^*_t)^2}$$

$L^*_0$, respectivement $a^*_0$, respectivement $b^*_0$ étant la valeur de L*, respectivement la valeur de a*, respectivement la valeur de b* mesurée avant le stockage et $L^*_t$, respectivement $a^*_t$, respectivement $b^*_t$ étant la valeur de L, respectivement la valeur de a, respectivement la valeur de b mesurée après un temps spécifique (t) de stockage et d'exposition ;

b) mesurer la valeur de L* et la valeur de a* et la valeur de b* du tocophérol de formule (I) correspondant qui n'a pas été mis en contact avec une terre décolorante activée par acide avant et après le temps spécifique (t) de stockage et d'exposition à l'air sélectionné à l'étape a) et en calculer la valeur $\Delta E^*_t$

$$\Delta E^*_t = \sqrt{(L^*_0 - L^*_t)^2 + (a^*_0 - a^*_t)^2 + (b^*_0 - b^*_t)^2}$$

$L^*_0$, respectivement $a^*_0$, respectivement $b^*_0$ étant la valeur de L*, respectivement la valeur de a*, respectivement la valeur de b* mesurée avant le stockage et $L^*_t$, respectivement $a^*_t$, respectivement $b^*_t$ étant la valeur de L*, respectivement la valeur de a*, respectivement la valeur de b* mesurée après un temps spécifique (t) de stockage et d'exposition ;

c) comparer la valeur $\Delta E^*_t$ mesurée à l'étape a) avec la valeur $\Delta E^*_t$ mesurée à l'étape b) ;

d) détecter que la valeur $\Delta E^*_t$ mesurée à l'étape a) est inférieure à la valeur $\Delta E^*_t$ mesurée à l'étape b) ; dans lequel les valeurs de L* et les valeurs de a* et les valeurs de b* sont mesurées selon la méthode CIE-L*a*b*.

10. Utilisation d'une terre décolorante activée par acide pour la décoloration de tocophérol de formule (I), sous la forme de tocophérol de formule (I) pur ou sous la forme d'une solution comprenant plus de 60 % en poids de tocophérol de formule (I),

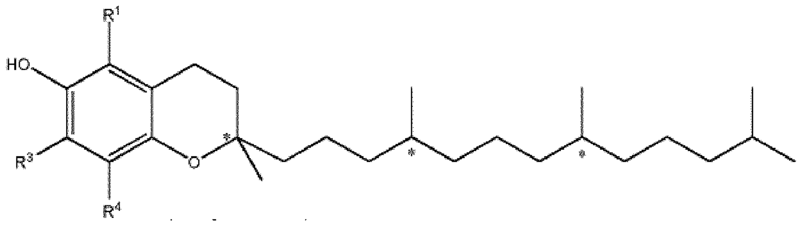

(I)

dans lequel $R^1$, $R^3$ et $R^4$ représentent indépendamment les uns des autres un hydrogène ou un groupe méthyle ; et dans lequel chaque * représente un centre chiral individuel.

**11.** Procédé de préparation d'un aliment ou d'une composition alimentaire avec une stabilité de couleur accrue comprenant les étapes consistant à

i) préparer un tocophérol de formule (I) avec une stabilité de couleur accrue selon le procédé des revendications 1 à 9 ; et
ii) ajouter le tocophérol de formule (I) avec une stabilité de couleur accrue issu de l'étape i) à au moins un complément alimentaire ou aliment ou ingrédient alimentaire.

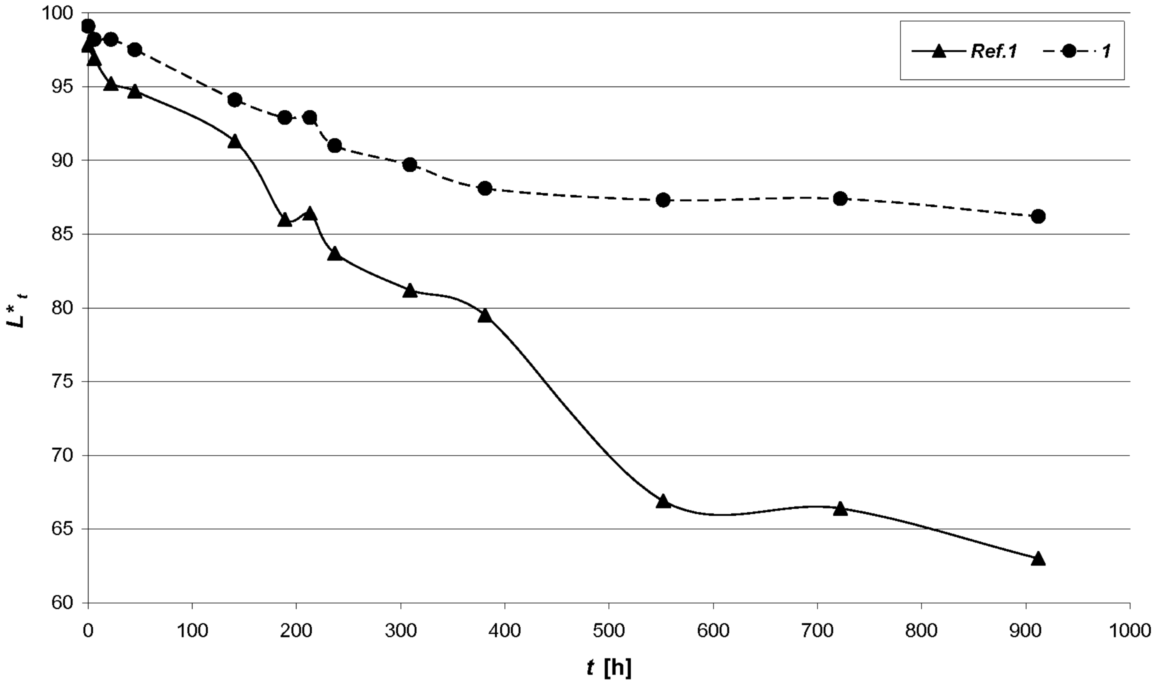


**Figure 1**

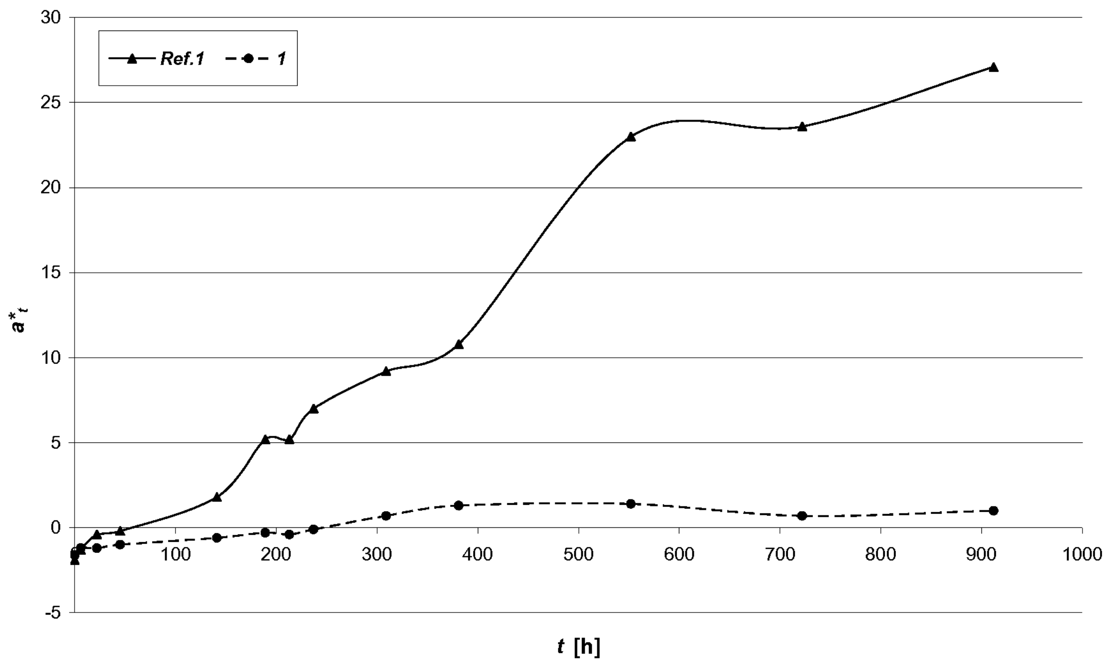


**Figure 2**

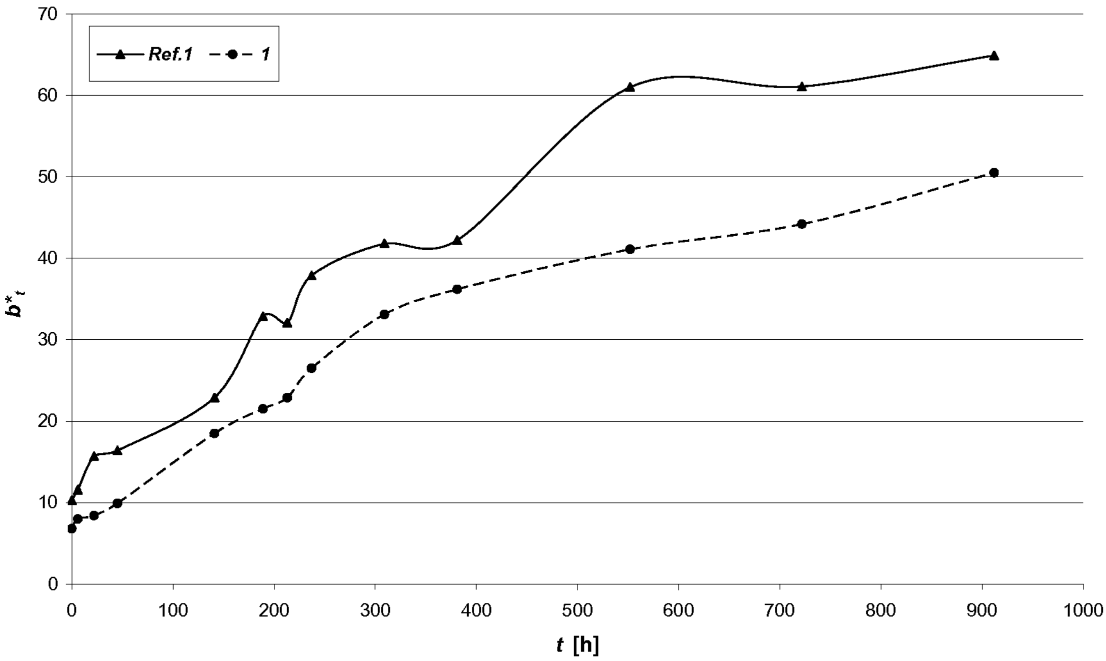


Figure 3

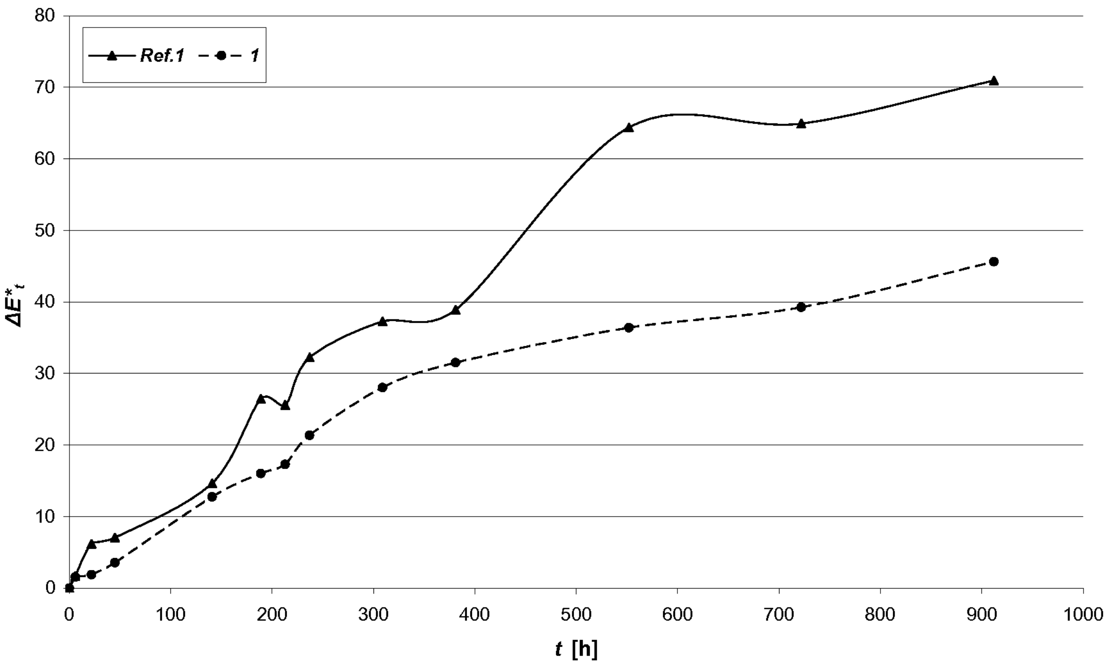


Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4101673 A
- FR 962797
- WO 2005054223 A2
- WO 2005121115 A1
- WO 2006066863 A1
- EP 11169784 A


### Non-patent literature cited in the description


- *J. Am. Oil Chem. Soc,* 2011, vol. 88, 127-132
- Lexikon der Chemie. **RÖMPP.** Bleicherde. 1996, 469
- Ullmann's Encyclopedia of Industrial Chemistry. Vitamins. 43-47